# EUROPÄISCHE PATENTSCHRIFT

(11) **EP 0 625 069 B1**
(45) Veröffentlichungstag und Bekanntmachung des Hinweises auf die Patenterteilung: **30.12.1998**
(21) Anmeldenummer: 93922486.1
(22) Anmeldetag: 18.10.1993
(51) Int. Cl.: B01J 13/02, B01J 13/12, A61K 9/16, A61K 9/50

(54) **VERFAHREN ZUR HERSTELLUNG VON MIKROKAPSELN**
METHOD OF MANUFACTURING MICROCAPSULES
PROCEDE DE FABRICATION DE MICROCAPSULES

(30) Priorität: 26.10.1992 CH 3319/92
(43) Veröffentlichungstag der Anmeldung: 23.11.1994
(73) Patentinhaber: SCHWARZ PHARMA AG, D-40789 Monheim/Rhld. (DE)
(72) Erfinder: GANDER, Bruno, CH-6405 Immensee (CH); MERKLE, Hans, Peter, CH-8049 Zürich (CH)
(74) Vertreter: Werner, Hans-Karsten, Dr.Dipl.-Chem.
(86) Internationale Anmeldenummer: CH9300246
(87) Internationale Veröffentlichungsnummer: WO9409898

(56) Entgegenhaltungen:
- EP-A- 0 102 265
- EP-A- 0 315 875
- EP-A- 0 330 180
- EP-A- 0 505 966
- DATABASE WPI Week 8522, Derwent Publications Ltd., London, GB; AN 85-130922 & JP,A,60 067 417 (KATOH) 17. April 1985
- J. PHARM. PHARMACOL. Bd. 40 , 16. Dezember 1987 Seiten 754 - 757 R. BODMEIER ET AL. 'PREPARATION OF BIODEGRADABLE POLY( )LACTIDE MICROPARTICLES USING A SPRAY-DRYING TECHNIQUE' in der Anmeldung erwähnt

## Beschreibung

Die vorliegende Erfindung betrifft ein Verfahren zur Herstellung von bioabbaubaren Mikrokapseln unter Verwendung von bioabbaubaren und toxikologisch unbedenklichen Lösungsmitteln. Der verwendete Begriff Mikrokapsel umfasst einfachheitshalber sowohl eigentliche Mikrokapseln als auch Mikrosphären und Mikropartikel gemäss folgenden Definitionen: Der Begriff Mikrokapseln beschreibt kugelförmige Partikel im Korngrössenbereich von 1-1000 µm, aufgebaut aus einem inneren Kern, der den Wirkstoff in flüssiger oder fester Form enthält, und der Kapselwand aus Polymer. Die Begriffe Mikrosphären, bzw. Mikropartikel beschreiben kugelförmige und nichtkugelförmige Partikel im Korngrössenbereich von 1 - 1000 µm, aufgebaut aus einer Polymermatrix, in welcher der Wirkstoff als sogenannte feste Lösung oder Suspension eingebettet vorliegt.

Biologisch hochaktive und empfindliche Wirkstoffe wie gewisse Arzneistoffe, Peptide, Proteine, Enzyme und Impfstoffe werden bevorzugt parenteral als Injektionslösung angewendet. Viele dieser Wirkstoffe besitzen jedoch eine kurze biologische Halbwertszeit oder sind in Lösung appliziert nicht sehr aktiv (Impfstoffe). Dies bedeutet, dass eine kontinuierliche oder pulsartige Wirkstoffzufuhr (Langzeitfreigabe) unabdingbare Voraussetzung für eine erfolgreiche therapeutische oder präventive Behandlung darstellt. Eine derartige, über einen längeren Zeitraum anhaltende Wirkstoffzufuhr kann dadurch erreicht werden, dass der Wirkstoff beispielsweise in ein bioabbaubares Freigabesystem eingebaut wird. Solche Freigabesysteme können in Form von Mikrokapseln oder Implantatstäbchen hergestellt werden. Bioabbaubare Mikrokapseln haben sich in der Vergangenheit besonders gut bewährt, da diese Partikel mit üblichen Injektionsnadeln parenteral leicht verabreicht werden können. Bekannte bioabbaubare Arzneistofffreigabesysteme basieren auf Polyestern der Milch- und Glykolsäure (D.H. Lewis, Controlled release of bioactive agents from lactide/glycolide polymers, in: Biodegradable polymers as drug delivery systems, M. Chasin, and R. Langer (Eds.), M. Dekker, New York, 1990, pp. 1-41).

Zur Herstellung von Mikrokapseln aus Polyestern, wie Poly(lactiden) und Poly(lactiden-co-glycoliden), sind hauptsächlich drei Verfahren bekannt: die Koazervation (Phasenseparation), z.B. durch Zugabe eines Nichtlösungsmittels, die Lösungsmittel-Abdampfung (solvent evaporation), oder -Extraktion (solvent extraction) aus einer O/W-Dispersion, und die Sprühtrocknung (R. Jalil et al., J. of Microencapsulation 7, 297-325, (1990)) . Allen diesen Verfahren ist gemeinsam, dass für die Auflösung des bioabbaubaren Polymers ein organisches Lösungsmittel eingesetzt werden muss, welches durch das Verfahren wieder grösstenteils entfernt wird. Da die totale Entfernung des Lösungsmittels aus dem Polymer jedoch nicht möglich ist, verbleiben in den Mikrokapseln immer Lösungsmittelrückstände, die in der Grössenordnung von 0.01 - 10 % liegen (G. Spenlehauer et al., Biomaterials 10, 557-563, (1989)); D.H. Lewis et al., PCT WO 89/03678). Die bisher hauptsächlich beschriebenen Lösungsmittel für die drei obgenannten Verfahren sind nicht bioabbaubare und z.T. sehr toxische oder umweltschädliche Substanzen (Methylenchlorid, Chloroform, Benzol, Tetrahydrofuran, Acetonitril, Fluorchlorkohlenwasserstoffe u.ä.), welche die Vorteile dieser bioabbaubaren Freigabesysteme kompromittieren.

So werden beispielsweise nach der US 5,066'436 Mikrokapseln durch Koazervation hergestellt, welche in besonderem Masse mit solchen organischen Lösungsmitteln belastet sind. Neben dem Lösungsmittel für das Polymer werden weitere organische Lösungsmittel für die eigentliche Phasentrennung, für die Aushärtung und das Waschen der Mikrokapseln eingesetzt. Zudem sind die so hergestellten Mikrokapseln relativ gross und agglomerieren schon während des Herstellungsverfahrens leicht, wodurch die Entfernung von Restlösungsmittel noch zusätzlich erschwert wird.

Die der Lösungsmittel-Abdampfung oder -Extraktion anhaftenden Nachteile liegen einerseits in den erwähnten toxikologisch nicht unbedenklichen Lösungsmittelrückständen der Mikrokapseln, andererseits in der Schwierigkeit, daß wasserlösliche Wirkstoffe wie gewisse Arzneistoffe, Peptide, Proteine, Enzyme und Impfstoffe durch die wäßrige Dispersionsphase herausgelöst werden und damit teilweise oder ganz der Verkapselung entgehen.

Die Sprühtrocknung ist ein bekanntes, einfaches und schnelles Verfahren zur Herstellung bioabbaubarer Mikrokapseln. Dabei wird jedoch immer wieder auf die Schwierigkeit hingewiesen, mit den bioabbaubaren Polymeren der Milch- und Glykolsäure sphärische und nicht poröse Partikel zu produzieren. Mit der in Methylenchlorid gelösten Poly(d,1-milchsäure) werden sehr unregelmäßig geformte Mikrokapseln mit einer unregelmäßigen Oberfläche und einem hohen Anteil an fiberähnlichem Material erhalten (R. Bodmeier et al., J. Pharm. Pharmacol. 40, 754-757 (1988)). Nach der EP A1 3151875 ist bekannt, daß die Copolymere der Milch- und Glykolsäure (PLGA) sich nicht mittels Sprühtrocknung zu Mikrokapseln verarbeiten lassen. Diese Copolymere sind jedoch als Verkapselungsmaterial bekannterweise von besonderem Interesse, da sie sich im Organismus schneller abbauen als die reinen Homopolymere der Milch-, bzw. Glykolsäure. Es wurde beschrieben, daß die PLGA-Polymere, insbesondere PLGA mit einem Anteil von je 50° Milch- und Glykolsäure [PLGA 50:50], eine Wirkstofffreisetzung über 3 - 4 Wochen ermöglichen, was beispielsweise für Hormon- und Enzymtherapie ein wünschenwertes Dosierungsintervall darstellt.

Die EP 0 505 966 A1 beschreibt langwirkende bioabbaubare Mikropartikel enthaltend therapeutisch wirksame Peptide als Wirkstoff und Poly(lactid-co-glycolid) als Trägermaterial, die durch Sprühtrocknung bei Temperaturen unter 60°C bei einem Sprühflow über 500 NL/h erhalten werden, wobei als Lösungsmittel bevorzugt Chloroform, Methylenchlorid, Eisessig, Methanol, Dimethylformamid oder Wasser verwendet wird.

Eine weitere Schwierigkeit bei der Mikroverkapselung mittels Sprühtrocknung ergibt sich aus den Eigenschaften der zu verkapselnden Substanz. Substanzen, die sich in der Polymerlösung (meist in Methylenchlorid) lösen lassen, werden gewöhnlich mit relativ hoher Effizienz und homogener Wirkstoffverteilung in die Mikrokapseln (hohe "content uniformity") eingebaut. Im Gegensatz dazu ist die Mikroverkapselung von Substanzen, die sich in der Polymerlösung nicht in Lösung bringen lassen, wie z.B. bestimmte Arzneistoffe, Peptide, Proteine, Enzyme und Impfstoffe, problematischer. Werden diese Wirkstoffe in mikronisierter Form in der organischen Polymerlösung suspendiert, wird oft nur eine unbefriedigende Einbaueffizienz erreicht, die sehr stark von der Feinheit des Wirkstoffes abhängt. Wasserlösliche Wirkstoffe werden deshalb oft zunächst in Wasser gelöst, und die wäßrige Wirkstofflösung darauffolgend in der Polymerlösung dispergiert (W/0-Dispersion). Mit den üblichen bekannten Lösungsmitteln können jedoch nur unbefriedigende W/0-Dispersionen hergestellt werden, die keine genügende Feinheit und physikalische Stabilität aufweisen. Die Folge davon ist ein schnelles Zusammenfließen der dispergierten, wäßrigen Phase (Koaleszenz), und damit verbunden eine schlechte Verkapselungseffizienz. Es wird deshalb immer wieder beobachtet, daß mikroverkapseltes Protein innerhalb von 1 - 3 Tagen freigegeben wird; dies wird im allgemeinen auf einen hohen Anteil an ungenügend verkapseltem Wirkstoff und hohe Mikrokapselporosität zurückgeführt (H. T. Wang et al., Biomaterials, 11, 679-685 (1990)). Außerdem können mit Wasser mischbare Lösungsmittel wie Aceton, Tetrahydrofuran, Dioxan, Acetonitril u.ä. nicht verwendet werden, da es wegen eben dieser Mischbarkeit zu Ausfällungen oder Aggregationen von Wirkstoff und/oder Polymer kommt.

Die Aufgabe der Erfindung wird dadurch gelöst, daß das bioabbaubare Polymer in einem bioabbaubaren und toxikologisch unbedenklichen Lösungsmittel gelöst wird, und die Wirkstoffe, resp. die biologisch aktiven Substanzen, wie Arzneistoffe, Peptide, Proteine, Enzyme und Impfstoffe in diese eingebracht werden, und durch Sprühtrocknen Mikrokapseln hergestellt werden.

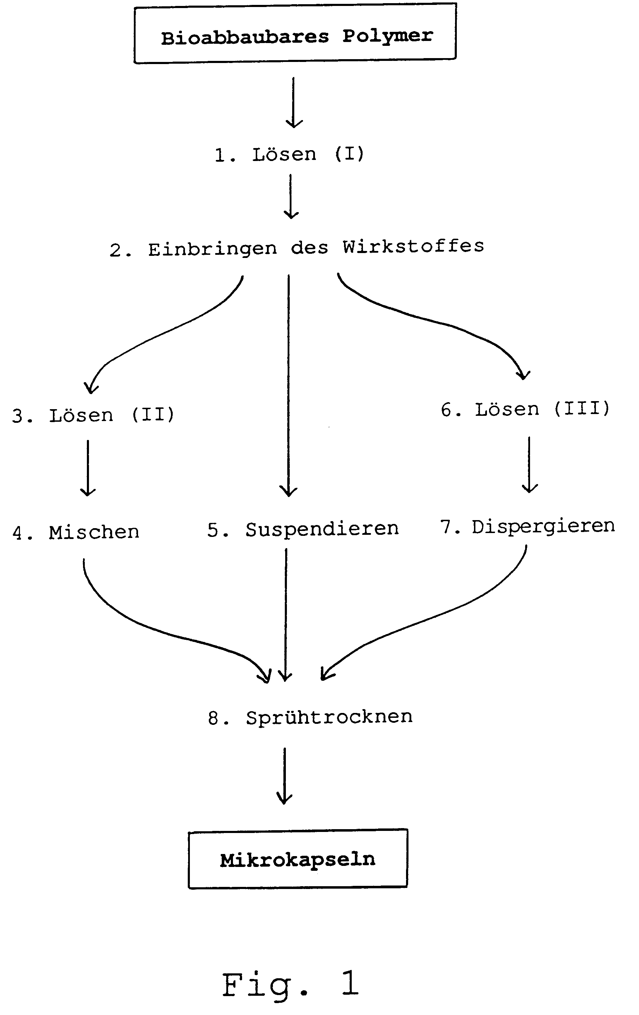

Erfindungsgemäß wird diese Aufgabe mit einem Verfahren gemäß dem Wortlaut der Patentansprüche 1 bis 6 gelöst. Das erfindungsgemäße Verfahren wird unter Bezug auf das Flussdiagramm (Fig. 1) näher erläutert, wobei in Fig. 1 die allgemeinen Schritte dargestellt sind. Dabei werden für die Beschreibung der verschiedenen Verfahrensschritte diejenigen Referenzzahlen des Flussdiagrammes verwendet, die für die Gliederung der einzelnen Schritte angeführt sind. In den Beispielen 1 - 5 werden Ausführungsbeispiele dazu beschrieben.
**Fig. 1** zeigt das Flussdiagramm für ein Verfahren zur Herstellung von Mikrokapseln, welches durch die Schritte 1 - 8 nachstehend beschrieben wird:

### 1. Lösen (I)

Ausgangspunkt des Verfahrens sind alle bioabbaubaren Polymere, soweit sie in den erfindungsgemässen Lösungsmitteln löslich sind, wie Mono-und Copolyester der Milch-, Glykol-, und β-Hydroxybuttersäure, sowie von δ-Valerolacton und ε-Caprolacton. Polymilch- und Polyglykolsäure werden auch als Poly(lactide) und Poly(glycolide) bezeichnet. Das Lösen des bioabbaubaren Polymers, bzw. eines Gemisches von bioabbaubaren Polymeren, erfolgt in den erfindungsgemässen Lösungsmitteln, wobei eine Polymerlösung (erste Lösung) entsteht. Zu diesen Lösungsmitteln gehört die Gruppe der einfachen Ester, aufgebaut aus einem C₁-C₅-Alkohol und einer C₁-C₅-Monocarbonsäure, sowie Gemische dieser einfachen Ester mit einem C₁-C₅-Alkohol. Zu diesen bioabbaubaren Lösungsmitteln gehören beispielsweise Ameisensäureethylester, -propylester, -butylester, Essigsäureethylester, -propylester, -isopropylester, Propionsäureethylester, Buttersäureethylester und Valeriansäureethylester, sowie Gemische dieser Ester mit Ethanol, Propanol, Isopropanol u.ä.. Diese Lösungsmittel, bzw. Lösungsmittelgemische, besitzen z.B. für die bioabbaubaren Polyesterverbindungen aus Glykol-, Milch-, β-Hydroxybuttersäure, δ-Valerolacton und aus ε-Caprolacton ein überraschend hohes Lösevermögen. Sie vermögen sowohl Homo- wie auch Copolymere dieser Hydroxycarbonsäuren in den unterschiedlichsten molaren Verhältnissen, wie etwa 50:50, 65:35, 75:25 oder 85:15 und in einem weiten Molekulargewichtsbereich (2000 - 150000) zu lösen. Dabei können die Polymere einzeln oder als Mischung in Lösung gebracht werden, und die erfindungsgemässen Lösungsmittel einzeln oder als Lösungsmittelgemisch, bestehend aus verschiedenen Estern oder aus Estern und Alkoholen, verwendet werden.

### 2. Einbringen des Wirkstoffes

Das Einbringen des zu verkapselnden Wirkstoffes in das bioabbaubare Polymer erfolgt in Abhängigkeit von dessen Löslichkeit nach drei Arten, entsprechend den Schritten 3, 5 und 6. Als Wirkstoffe werden nachfolgend Substanzen definiert, die in lebenden Organismen eine biologische Wirkung entfalten, wobei diese beispielsweise pharmakologischer, immunologischer oder enzymatischer Art sein kann. Der zu verkapselnde Wirkstoff liegt im allgemeinen in fester Form (Pulver) vor, kann aber auch in flüssiger Form vorliegen und als Flüssigkeit verarbeitet werden.

### 3. Lösen (II)

Wirkstoffe, die sich in den erfindungsgemässen bioabbaubaren Lösungsmitteln, bestehend aus Estern, aus C₁-C₅-Alkoholen und beliebigen Ester-Alkoholgemischen davon, lösen lassen, werden direkt darin gelöst, wobei eine organische Wirkstofflösung (zweite Lösung) entsteht. Diese Wirkstofflösung stellt den bevorzugten Verfahrensschritt dar, da sie mit minimalstem Aufwand hergestellt werden kann und thermodynamisch stabil ist.

### 4. Mischen

Anschliessend werden die Polymerlösung (erste Lösung) und die organische Wirkstofflösung (zweite Lösung) zusammengegeben und gemischt, wodurch das Substrat 'Lösung' erhalten wird. Die hier beschriebenen Lösungsmittel zeichnen sich durch ein hohes Lösungsvermögen für eine Vielzahl niedermolekularer Wirkstoffe, wie gewisse Arzneistoffe und Peptide, aus. Diese vorteilhafte Eigenschaft dieser Lösungsmittel ermöglicht es auch, den Wirkstoff und das bioabbaubare Polymer gleichzeitig in Lösung zu bringen, indem dabei das gleiche Lösungsmittel oder eine Kombination von bioabbaubaren Lösungsmitteln verwendet wird. Das Verfahren wird mit Schritt 8 fortgesetzt.

### 5. Suspendieren

Wirkstoffe, die sich weder in Wasser noch in den erfindungsgemässen Lösungsmitteln genügend lösen lassen, werden in der Polymerlösung, die das bioabbaubare Polymer in den bioabbaubaren Lösungsmitteln enthält, mechanisch oder mittels Ultraschall suspendiert, wodurch das Substrat 'Suspension' erhalten wird. Um eine hohe Einbaueffizienz zu erreichen, wird der Wirkstoff vorzugsweise in mikronisierter Form vorgelegt. Dabei werden für den Wirkstoff im allgemeinen Partikel kleiner 10 µm angestrebt. Das Verfahren wird mit Schritt 8 fortgesetzt.

### 6. Lösen (III)

Gut wasserlösliche Wirkstoffe, wie bestimmte Arzneistoffe, Peptide, Proteine, Enzyme, oder Impfstoffe, die in den erfindungsgemässen Lösungsmitteln nicht gelöst werden können, werden vorteilhafterweise in einem möglichst kleinen Volumen eines wässrigen Mediums gelöst, wobei eine wässrige Wirkstofflösung entsteht. Das wässrige Medium kann neben Wasser Zusätze enthalten, wie beispielsweise Puffersalze, Tenside, Stabilisatoren und Konservierungsmittel.

### 7. Dispergieren

Die nach Schritt 6 erhaltene wässrige Wirkstofflösung wird darauffolgend in der Polymerlösung, die das bioabbaubare Polymer in den erfindungsgemässen Lösungsmitteln enthält, feinst dispergiert, wodurch das Substrat 'W/O-Dispersion' erhalten wird. Dabei lassen sich wässrige Wirkstofflösungen bis zu einem Volumenanteil von 50% und unter wenig Energieaufwand in die Polymerlösung nanodispers einarbeiten. Die Dispergierung kann durch einfaches Schütteln, besser jedoch mittels mechanischem Dispergiergerät oder Ultraschall erfolgen. Die bevorzugte Dispergiermethode ist die Ultrabeschallung mit 50 - 400 W während 10 - 300 s oder nötigenfalls auch länger. Diese Dispersionen von wässriger Wirkstofflösung in Polymerlösung bleiben überraschenderweise auch ohne Zusatz von Tensiden oder Pseudoemulgatoren über mehrere Stunden stabil (keine Koaleszenz) und müssen während des Sprühprozesses nicht kontinuierlich dispergiert werden, was sich als besonders vorteilhaft erweist. Die erfindungsgemässen Lösungsmittel ergeben mit wässrigen Wirkstofflösungen W/O-Dispersionen, die bezüglich Feinheit und Stabilität mit den üblicherweise verwendeten Lösungsmitteln, wie beispielsweise Methylenchlorid, nicht erreicht werden können. Das Verfahren wird mit Schritt 8 fortgesetzt.

### 8. Sprühtrocknen

Die nach den Schritten 4, 5 und 7 verfügbaren Substrate 'Lösung', 'Suspension' und 'W/O-Dispersion' werden durch Sprühtrocknen zu Mikrokapseln verarbeitet, was beispielsweise mit einem Mini Spray Dryer 190 (Büchi) erfolgt. Durch Sprühtrocknen hergestellte Mikrokapseln haben im allgemeinen Durchmesser von 1 - 50 µm, maximal jedoch 100 µm, und können damit noch leicht eine Injektionsnadel passieren. Der Beladungsgrad der Mikrokapseln (Wirkstoffgehalt) beträgt zwischen 0 - 50%, vorzugsweise jedoch zwischen 0 - 20%.

Die auf diese Weise hergestellten Mikrokapseln werden zweckmässig - und dem Endverbrauch angepasst - weiterverarbeitet, wobei beispielsweise ein Waschprozess, eine Fraktionierung in unterschiedliche Korngrössen, eine Endtrocknung unter Hochvakuum, eine Sterilisation mittels γ-Bestrahlung oder beliebige weitere Prozesse folgen können.

Das hier mit den Schritten 1 - 8 beschriebene Verfahren ermöglicht aufgrund des hohen Lösungsvermögens der erfindungsgemässen Lösungsmittel für viele Wirkstoffe und aufgrund der leichten Dispergierbarkeit von wässrigen Wirkstofflösungen eine hohe Einbaueffizienz und eine homogene Wirkstoff-in-Polymer Verteilung für Wirkstoffe mit unterschiedlichsten Löslichkeitseigenschaften. Aus den Mikrokapseln wird der Wirkstoff in Abhängigkeit des verwendeten Polymers, des Beladungsgrades und eventueller Zusatzstoffe (Freigaberegulatoren) über einen Zeitraum von 1 Tag bis 1 Jahr freigegeben.

Im Gegensatz zu den üblicherweise verwendeten halogenierten und nichthalogenierten organischen Lösungmitteln ermöglichen es die erfindungsgemässen bioabbaubaren Lösungsmittel, auch mit Poly(d,l-milchsäure) oder Poly(milch-co-glykolsäure) Mikrokapseln durch Sprühtrocknung herzustellen. Die erhaltenen Produkte zeigen eine regelmässig-kugelförmige Partikelmorphologie und eine glatte nichtporöse Oberfläche. Partikelverklebungen, -agglomerationen oder -verformungen werden kaum beobachtet.

Die erfindungsgemässen Lösungsmittel besitzen überdies den ausserordentlichen Vorteil, dass sie toxikologisch unbedenklich sind. Genau wie die den Mikrokapseln zugrundeliegenden Polymere bauen sich die erfindungsgemässen Lösungsmittel im Organismus in kleinere, untoxische, und z.T. physiologische Bestandteile ab. Dieser Abbau geschieht im wesentlichen durch Hydrolyse, kann jedoch auch enzymatisch, beispielsweise durch Esterasen oder Dehydrogenasen, erfolgen. Im Gegensatz zu den erfindungsgemässen Lösungsmitteln werden die sonst üblicherweise verwendeten Lösungsmittel im Organismus zu toxischen Metaboliten umgeformt. Nebst dem begründeten Verdacht auf ein krebserzeugendes Potential und unterschiedlichen toxischen Effekten entsteht aus Methylenchlorid im Organismus beispielsweise das Atemgift Kohlenstoffmonoxid.

**Beispiel 1** beschreibt die Herstellung von Mikrokapseln, enthaltend ein Protein: 0,09 g Rinderserumalbumin (Fluka, CH-Buchs) wurden in 2,16 g Wasser gelöst. Die wässrige Lösung wurde mit Hilfe eines Ultraschallgenerators in einer Lösung von 3,0 g Poly(d,l-milchsäure) (Resomer R202, Boehringer Ingelheim) in 57,0 g Essigsäureisopropylester dispergiert. Die Dispergierung erfolgte unter Eiskühlung in einem Ultraschallprozessor (Vibracell, VC375, Sonics and Materials, Danbury, CT) mit einer Energie von 260 W während 2 x 30 s. Die Dispersion wird in einem Laborsprühtrockner (Mini Spray Dryer, Büchi 190, Büchi Laboratorien, CH-Flawil) unter folgenden Bedingungen versprüht: Eingangstemperatur: 55 °C, Sprühflow: 400 Skalenteile, Aspirator: 15 Skalenteile, Sprühgeschwindigkeit: 2,4 ml/min.

Die Mikrokapseln fallen als weisses, freifliessendes Pulver an. Ausbeute: 1,8 g (58% der Theorie), Wirkstoffgehalt: 2,9% (100% der Theorie).

**Beispiel 2** beschreibt die Herstellung von Mikrokapseln, enthaltend ein Protein: 0,09 g Rinderserumalbumin wurden in 2,16 g Wasser gelöst. Die wässrige Lösung wurde mit Hilfe eines Ultraschallgenerators (260 W während 30 s) in einer Lösung von 3,0 g Poly(d,l-milchsäure) (Resomer R202, Boehringer Ingelheim) in 57,0 g Essigsäureethylester dispergiert. Dispergierung und Sprühtrocknung erfolgten unter identischen Bedingungen wie in Beispiel 1. Ausbeute: 1,6 g (52% der Theorie), Wirkstoffgehalt: 2,9% (100% der Theorie).

**Beispiel 3** beschreibt die Herstellung von Mikrokapseln, enthaltend einen Impfstoff: 0,100 g lyophilisiertes Tetanus Toxoid wurde in 2,0 ml Wasser gelöst. Die wässrige Lösung wurde mit Hilfe eines Ultraschallgenerators in einer Lösung von 5,0 g Poly(d,l-milch-säure-co-glykolsäure) (Resomer RG502, Boehringer Ingelheim) in 100,0 g Ameisensäureethylester dispergiert. Die Dispergierung erfolgte unter Eiskühlung mit einer Ultraschallenergie von 210 W während 2 x 30 s. Die Dispersion wurde in einem Laborsprühtrockner unter folgenden Bedingungen versprüht: Eingangstemperatur: 55 °C, Sprühflow: 500 Skalenteile, Aspirator: 17,5 Skalenteile, Sprühgeschwindigkeit: 2,4 ml/min.

Die Mikrokapseln fielen als weisses, freifliessendes Pulver an. Ausbeute: 0,7 g (40% der Theorie), Proteingehalt: 1,82%, entsprechend 93% des theoretisch ermittelten Wirkstoffgehalts.

**Beispiel 4** beschreibt die Herstellung von Mikrokapseln, enthaltend einen Enzym: 15 mg Peroxidase aus Meerrettich mit einer Aktivität von 75,8 U/mg (Fluka, CH-Buchs) werden in 0,6 ml Wasser gelöst. Die wässrige Lösung wird mittels Ultraschall von 375 W während 30 s unter Eiskühlung in einer Lösung von 1,5 g Poly(d,l-milchsäure) (Resomer R202, Boehringer Ingelheim) in 30,0 g Ameisensäureethylester dispergiert. Die Dispersion wird mit einem Laborsprühtrockner unter folgenden Bedingungen versprüht: Eingangstemperatur: 47 °C, Sprühflow: 450 Skalenteile, Aspirator: 17 Skalenteile, Sprühgeschwindigkeit: 1,6 ml/min.

Die Mikrokapseln fallen als weisses, freifliessendes Pulver an. Ausbeute: 0,92 g (61% der Theorie), Enzymaktivität: 0,177 U/mg Mikrokapseln, entsprechend 23,4% der theoretisch ermittelten Aktivität.

**Beispiel 5** beschreibt die Herstellung von Mikrokapseln, enthaltend einen Arzneistoff: 30 mg Prostaglandin E2 (Fluka, CH-Buchs) und 6,0 g Poly(d,1-milchsäure-co-glykolsäure) (Resomer RG502, Boehringer Ingelheim) wurden getrennt in je 60 g Ameisensäureethylester gelöst. Die vereinten Lösungen wurden unter folgenden Bedingungen versprüht: Eingangstemperatur: 45°C, Sprühflow: 420 Skalenteile, Aspirator: 15 Skalenteile, Sprühgeschwindigkeit: 2,4 ml/min.

Die Mikrokapseln fallen als weisses, freifliessendes Pulver an. Ausbeute: 4,2 g (70% der Theorie), Beladungsgrad: 4,99 µg/mg Mikrokapseln (100% der Theorie).

**Beispiel 6** beschreibt die Herstellung von Mikrokapseln, enthaltend ein Hormon: 25 g 17-β-Estradiol werden in einer Mischung aus 125 ml Ethanol wasserfrei und 312,5 ml Essigsäureethylester gelöst. 100 g Resomer RG 503 (Boehringer Ingelheim) werden in 812,5 ml Ameisensäureethylester gelöst. Beide Lösungen werden vor dem Versprühen unter Rühren und Bildung einer stabilen Suspension vereinigt.

Die Suspension wird unter folgenden Bedingungen in einem mobilen Minor Spray Dryer (Fa. NIRO AS, Kopenhagen) versprüht: Eingangstemperatur: 67 °C, Sprühgeschwindigkeit: 0,9 l/h.

Die Mikrokapseln fallen als weisses Pulver an, enthaltend diskrete, sphärische Partikel. Ausbeute: 80 g (80% der Theorie); Beladungsgrad: 70,2 µg/mg Mikrokapseln (85,1% der Theorie).

**Beispiel 7** beschreibt die Herstellung von Mikrokapseln, enthaltend ein Hormon: 10 g 17-β-Estradiol werden in einer Mischung aus 100 ml Ethanol wasserfrei und 250 ml Essigsäureethylester gelöst. 90 g Resomer RG 752 (Boehringer Ingelheim) werden in 650 ml Ameisensäureethylester gelöst. Die beiden Lösungen werden unter Rühren vereinigt und unter folgenden Bedingungen in einem mobilen Minor Spray Dryer (Fa. NIRO AS, Kopenhagen) versprüht: Eingangstemperatur: 70 °C, Sprühgeschwindigkeit: 0,9 l/h.

Die Mikrokapseln fallen als weisses Pulver an, enthaltend diskrete, sphärische Partikel. Ausbeute: 100 g (85% der Theorie); Beladungsgrad: 89,6 µg/mg Mikrokapseln (89,6% der Theorie).

**Beispiel 8** beschreibt die Herstellung von Mikrokapseln, enthaltend einen synthetischen Impfstoff: 0,02 g eines synthetischen Proteins, bestehend aus der Peptidsequenz 947-967 des Tetanus Toxins und einem B-Zell Epitop der repetitiven Region des Circumsporozoit Proteins von Plasmodium berghei, wurde in 1,0 ml Wasser gelöst. Die wässrige Lösung wurde mit Hilfe eines Ultraschallgenerators in einer Lösung von 2,0 g Poly(d,l-milchsäure-co-glykolsäure) (Resomer RG 752, Boehringer Ingelheim) in 40,0 g Ameisensäureethylester dispergiert. Die Dispergierung erfolgte unter Eiskühlung mit einer Ultraschallenergie von 210 W während 2 x 30 s. Die Dispersion wurde in einem Laborsprühtrockner unter folgenden Bedingungen versprüht: Eingangstemperatur: 50 °C, Sprüh-Flow: 450 Skalenteile, Aspirator: 14 Skalenteile, Sprühgeschwindigkeit: 2,6 ml/min.

Die Mikrokapseln fallen als weisses, freifliessendes Pulver an. Ausbeute: 1,12 g (56% der Theorie); Proteingehalt: 0,66%, entsprechend 0,6% des theoretisch ermittelten Wirkstoffgehaltes.

Erfindungswesentlich ist, dass biologisch abbaubare Lösungsmittel vorgeschlagen werden, womit die damit hergestellten Mikrokapseln frei von toxischen Restlösungsmitteln sind. Die Mikrokapseln werden durch Versprühen einer Lösung, Suspension oder Dispersion aus Wirkstoffen und Polymeren erhalten. Als einzubettende Wirkstoffe eignen sich sowohl wasserlösliche wie auch wasserunlösliche Substanzen. Wasserlösliche Wirkstoffe wie Peptide, Proteine, Impfstoffe werden vorzugsweise als wässrige Lösung in der Polymerlösung dispergiert. Hiermit steht ein Verfahren zur Verfügung, welches in der Herstellung von Arzneistoff-, Impfstoff- und Enzymzubereitungen Anwendung findet.

## Patentansprüche

1. Verfahren zur Herstellung von Mikrokapseln, die einen biologisch aktiven Wirkstoff enthalten und die aus einem physiologisch verträglichen, im lebenden Organismus abbaubaren Polymeren bestehen, durch
a) Herstellen einer Lösung des physiologisch verträglichen, im lebenden Organismus abbaubaren Polymeren,
b) Einbringen der Wirksubstanz durch
- Auflösen oder Vermischen einer Lösung der Wirksubstanz in einem mit dem im Schritt a) verwendeten Lösungsmittel mischbaren Lösungsmittel, oder
- Suspendieren des mikronisierten Wirkstoffes, oder
- Dispergieren einer wäßrigen Lösung des Wirkstoffes in der in Schritt a) hergestellten Lösung unter Bildung einer W/O-Dispersion,
in die in Schritt a) hergestellte Lösung des physiologisch verträglichen, im lebenden Organismus abbaubaren Polymeren, und
c) Sprühtrocknen der in Schritt b) erhaltenen Lösung, Suspension oder W/O-Dispersion,
dadurch gekennzeichnet, daß als Lösungsmittel zum Lösen des physiologisch verträglichen, im lebenden Organismus abbaubaren Polymeren und gegebenenfalls der Wirksubstanz mindestens ein C₁-C₅-Alkylester einer C₁-C₅-Monocarbonsäure gegebenenfalls im Gemisch mit mindestens einem C₁-C₅-Alkohol verwendet wird und die Sprühtrocknung bei einer Temperatur von 40 bis 70 °C durchgeführt wird unter Bedingungen, die einem Sprühflow von 400 bis 500 NL/h bei einem Mini-Spray Dryer Büchi 190 entsprechen.

2. Verfahren nach Anspruch 1, dadurch gekennzeichnet, daß als Lösungsmittel Ameisensäureethylester, Essigsäureethylester oder Essigsäureisopropylester verwendet wird.

3. Verfahren nach einem der Ansprüche 1 oder 2, dadurch gekennzeichnet, daß als Lösungsmittel eine Mischung aus Essigsäureethylester und Ethanol verwendet wird.

4. Verfahren nach einem der Ansprüche 1 bis 3, dadurch gekennzeichnet, daß als Wirkstoff ein Arzneistoff, ein Peptid, ein Protein, ein Enzym oder ein Impfstoff verwendet wird.

5. Verfahren gemäß Anspruch 4, dadurch gekennzeichnet, daß der Wirkstoff Prostaglandin E2 ist.

6. Verfahren gemäß Anspruch 4, dadurch gekennzeichnet, daß der Wirkstoff 17-β-Estradiol ist.

## Claims

1. A process for the preparation of microcapsules containing a biologically active substance and consisting of a physiologically compatible polymer which is degradable in the living organism, by
a) preparing a solution of said physiologically compatible polymer which is degradable in the living organism;
b) introducing the active substance into the solution of said physiologically compatible polymer which is degradable in the living organism as prepared in step a) by
- dissolving the active substance in a solvent miscible with the solvent used in step a) and mixing the two solutions; or
- suspending the active substance in a micronized state; or
- dispersing an aqueous solution of the active substance in the solution prepared in step a) to form a w/o dispersion; and
c) spray-drying the solution, suspension or w/o dispersion obtained in step b);
characterized in that at least one C₁-C₅-alkyl ester of a C₁-C₅-monocarboxylic acid, optionally in admixture with at least one C₁-C₅-alcohol, is used as the solvent for dissolving said physiologically compatible polymer which is degradable in the living organism and said active substance, if dissolved in a solvent, and said spray-drying is performed at a temperature of from 40 to 70 °C under conditions which correspond to a spray flow of 400 to 500 NL/h as set on a Mini Spray Dryer Büchi 190.

2. The process according to claim 1, characterized in that ethyl formiate, ethyl acetate or isopropyl acetate is used as the solvent.

3. The process according to any of claims 1 or 2, characterized in that a mixture of ethyl acetate and ethanol is used as the solvent.

4. The process according to any of claims 1 to 3, characterized in that a medicament, a peptide, a protein, an enzyme or a vaccine is used as said active substance.

5. The process according to claim 4, characterized in that said active substance is prostaglandin E2.

6. The process according to claim 4, characterized in that said active substance is 17-β-estradiol.

## Revendications

1. Procédé de fabrication de microcapsules qui contiennent une substance biologiquement active et qui sont constituées d'un polymère compatible d'un point de vue physiologique, dégradable dans l'organisme vivant,
a) par préparation d'une solution du polymère compatible d'un point de vue physiologique, dégradable dans l'organisme vivant,
b) par introduction de la substance active
- par dissolution ou mélange d'une solution de la substance active dans un solvant miscible au solvant utilisé dans l'étape a),ou
- par mise en suspension de la substance active micronisée, ou
- par dispersion d'une solution aqueuse de la substance active dans la solution préparée dans l'étape a), avec formation d'une dispersion eau dans l'huile,
dans la solution préparée dans l'étape a) du polymère compatible d'un point de vue physiologique, dégradable dans l'organisme vivant, et
c) par séchage par atomisation de la solution, de la suspension ou de la dispersion eau dans l'huile obtenue dans l'étape b),
caractérisé en ce qu'on utilise en tant que solvant pour dissoudre le polymère compatible d'un point de vue physiologique, dégradable dans l'organisme vivant, et éventuellement la substance active, au moins un ester d'alkyle en C₁-C₅ d'un acide monocarboxylique en C₁-C₅, éventuellement en mélange avec au moins un alcool en C₁-C₅, et que l'on met en oeuvre le séchage par atomisation à une température de 40 à 70 °C, dans des conditions correspondant à un débit d'atomisation de 400 à 500 litres normaux/h, sur un sécheur par atomisation Mini-Spray Dryer Büchi 190.

2. Procédé selon la revendication 1, caractérisé en ce qu'on utilise en tant que solvant le formiate d'éthyle, l'acétate d'éthyle ou l'acétate d'isopropyle.

3. Procédé selon l'une des revendications 1 ou 2, caractérisé en ce qu'on utilise en tant que solvant un mélange d'acétate d'éthyle et d'éthanol.

4. Procédé selon l'une des revendications 1 à 3, caractérisé en ce qu'on utilise, en tant que substance active, un médicament, un peptide, une protéine, une enzyme ou un inoculant.

5. Procédé selon la revendication 4, caractérisé en ce que la substance active est la prostaglandine E2.

6. Procédé selon la revendication 4, caractérisé en ce que la substance active est le 17-β-oestradiol.
